# EUROPÄISCHE PATENTSCHRIFT

(11) **EP 0 944 832 B1**
(45) Veröffentlichungstag und Bekanntmachung des Hinweises auf die Patenterteilung: **08.08.2001**
(21) Anmeldenummer: 97953700.8
(22) Anmeldetag: 26.11.1997
(51) Int. Cl.: G01N 33/53

(54) **ANTIGENSPEZIFISCHER IgM-NACHWEIS**
ANTIGEN-SPECIFIC IgM DETECTION
DETECTION D'IgM SPECIFIQUES D'ANTIGENES

(30) Priorität: 29.11.1996 DE 19649389
(43) Veröffentlichungstag der Anmeldung: 29.09.1999
(73) Patentinhaber: Roche Diagnostics GmbH, 68298 Mannheim (DE)
(72) Erfinder: FAATZ, Elke, D-82386 Huglfing (DE); SCHMITT, Urban, D-82386 Oberhausen (DE); OFENLOCH-HÄHNLE, Beatus, D-82398 Polling (DE)
(86) Internationale Anmeldenummer: EP9706583
(87) Internationale Veröffentlichungsnummer: WO9823955

(56) Entgegenhaltungen:
- EP-A- 0 261 493
- EP-A- 0 280 211
- EP-A- 0 292 810

## Beschreibung

Die Erfindung betrifft ein Verfahren zur Bestimmung von antigenspezifischen Antikörpern der Immunglobulinklasse M in Körperflüssigkeiten durch Inkubation der Probe mit mindestens zwei verschiedenen Rezeptoren R₁ und R₂, wobei beide Rezeptoren mit dem Antikörper spezifisch bindefähig sind, R₁ an eine feste Phase gebunden ist oder gebunden werden kann und R₂ eine Markierung trägt, wobei ein wesentlicher Bestandteil von R₁ und gegebenenfalls auch von R₂ jeweils ein vom zu bestimmenden Antikörper spezifisch erkannter Bindepartner in polymerer Form ist und zur Entstörung von IgG-Antikörpern gleicher Spezifität Bindepartner in monomerer Form eingesetzt werden.

Insbesondere betrifft die Erfindung eine Methode zum spezifischen Nachweis von Immunglobulinen der Klasse IgM in Anwesenheit von Immunglobulinen der Klasse IgG und Störfaktoren wie beispielsweise Rheumafaktoren.

Das Immunsystem eines Säugetierorganismus produziert als Antwort auf das Einbringen fremder Substanzen Antikörper, die auch Immunglobuline genannt werden. Sie dienen zur Abwehr der Fremdsubstanzen, die auch als Antigene bezeichnet werden. Die Immunglobuline können in fünf verschiedene Klassen eingeteilt werden. Man unterscheidet Immunglobuline der Klassen M, G, A, E und D. Diese fünf Immunglobulinklassen unterscheiden sich jeweils in der Zusammensetzung der schweren Kette, die als µ-, γ-, α-, ε- bzw. δ-Kette bezeichnet wird.

Jede Immunglobulinklasse hat im Organismus eine andere Aufgabe. Die Immunglobuline der Klasse M treten bei einem ersten Kontakt mit dem Antigen, der sogenannten Erstimmunisierung, auf. Die Konzentration dieser Immunglobuline nimmt jedoch bei fortschreitender Infektion schnell wieder ab. Die Immunglobuline der Klasse G werden bei einer ersten Immunisierung erst langsam gebildet und treten bei einer zweiten Infektion mit demselben Antigen in großen Mengen auf. Die Immunglobuline der Klasse A sind auf den Schleimhautoberflächen des Organismus anzutreffen und sind für die dortigen Abwehrvorgänge zuständig. Die Immunglobuline der Klasse E sind hauptsächlich für allergische Reaktionen verantwortlich. Die genaue Funktion der Immunglobuline der Klasse D ist bislang nicht bekannt.

Die einzelnen Immunglobulinklassen kommen im Blut in sehr unterschiedlichen Konzentrationen vor. So sind die Immunglobuline der Klasse G (IgG) in normalem menschlichen Serum mit einem Anteil von etwa 75 %, das entspricht einem Serumgehalt von 8 bis 18 mg/ml, die am stärksten vertretene Klasse. Das am zweithäufigsten auftretende Immunglobulin ist das IgA, dessen durchschnittliche Serumkonzentration 0,9 bis 4,5 mg/ml beträgt. Immunglobuline der Klasse M sind in einer Konzentration von 0,6 bis 2,8 mg/ml, Immunglobuline der Klasse D von 0,003 bis 0,4 mg/ml vorhanden. Am geringsten ist der Anteil von IgE-Antikörpern, die nur in einer Konzentration von 0,02 bis 0,05 µg/ml im Serum auftreten.

Für die differenzierte Diagnostik vieler Erkrankungen ist es wichtig, den Nachweis für Antikörper einer oder mehrerer ganz bestimmter Immunglobulinklassen, die für ein bestimmtes Antigen spezifisch sind, zu führen. Nur mittels eines klassenspezifischen Antikörper-Nachweises, bzw. durch das Ausschließen der Anwesenheit bestimmter Immunglobulinklassen (z.B. Detektion von IgG- und IgA-Antikörpern, aber keine Detektion von IgM-Antikörpern) kann eine befriedigende Diagnose bei viralen, bakteriellen und parasitären Infektionen sichergestellt werden. Dies ist besonders wichtig zur Unterscheidung frischer bzw. akuter und weiter zurückliegender Infektionen sowie zur klinischen Kontrolle des Verlaufs einer Infektion. Der klassenspezifische Nachweis von Antikörpern ist insbesondere wichtig bei HIV-, Hepatitis A-, Hepatitis B-, Toxoplasmose-, Rubella- und Chlamydia-Infektionen. Ebenso ist der klassenspezifische Nachweis von für ein bestimmtes Antigen spezifischen Antikörpern bei der Bestimmung des Titers schützender Antikörper und zur Überprüfung des Impferfolges notwendig. Gerade für die Diagnostik frischer, akuter Infektionen ist es von besonderem Interesse, Antikörper der Klasse IgM, die für ein Antigen spezifisch sind, nachzuweisen. Häufig wird der Nachweis von antigenspezifischen IgM-Antikörpern jedoch durch verschiedene Störeinflüsse wie beispielsweise die Gegenwart von IgG-Antikörpern gleicher Spezifität gestört.

Im Stand der Technik werden verschiedene Methoden zum Nachweis von für ein Antigen spezifischen Antikörpern einer bestimmten Klasse beschrieben. So wird der Nachweis von antigenspezifischen Antikörpern einer bestimmten Klasse in einer Probe häufig dadurch geführt, daß die spezifischen Antikörper an eine mit dem spezifischen Antigen beschichtete Festphase gebunden werden. Der Nachweis der für das Antigen spezifischen, nun an die Festphase gebundenen Immunglobuline (Ig) erfolgt durch die Bindung von Antikörpern, die spezifisch gegen humanes Ig einer bestimmten Klasse gerichtet sind, an die nachzuweisenden Ig-Moleküle. Die gegen humanes Ig gerichteten Antikörper sind mit einer Markierung versehen, über die die Detektion stattfindet. Eine solche Testführung ist aber nur möglich, wenn vor der Reaktion mit den klassenspezifischen, gegen humanes Ig gerichteten markierten Antikörpern durch Waschung alles unspezifische, nicht gebundene Ig entfernt wird. Eine Einschritt-Testführung, wie sie für automatisierte Systeme oft benötigt wird, ist somit nicht möglich. Außerdem binden im ersten Schritt Antikörper aller Klassen, die spezifisch für das Antigen sind, an die Festphase. Ist die Antigenbeschichtung der Festphase nicht ausreichend hoch, kann es zu Konkurrenzreaktionen der verschiedenen Antikörperklassen um die Bindung an das Antigen kommen. Dadurch kann die Sensitivität des Tests leiden.

Eine Möglichkeit, einen Antikörper-Nachweis in einem Einschritt-Test durchzuführen, eröffnet der sogenannte Brückentest. Das Brückentest-Konzept ist in der EP-A-0 280 211 beschrieben. Dabei wird ein erster Rezeptor, der mit dem zu bestimmenden Antikörper spezifisch bindefähig ist wie beispielsweise ein Antigen, an eine Festphase gebunden. Der zu bestimmende Antikörper bindet an das festphasengebundene Antigen. Im Testansatz ist außerdem ein weiteres, spezifisches Antigen vorhanden, das mit einer Markierung versehen ist. Der Nachweis des Antikörpers erfolgt über die Markierung. Bei diesem Test werden jedoch alle antigenspezifischen Antikörper nachgewiesen und nicht nur die Antikörper einer bestimmten Klasse.

Ein weiterer Störeinfluß bei der Bestimmung von antigenspezifischen IgM-Antikörpern geht von Rheumafaktoren aus. Rheumafaktoren sind zumeist selbst Antikörper der Klasse IgM, die im allgemeinen eine hohe Affinität zu den Fc-Bereichen von IgG-Antikörpern haben. Dadurch ist es möglich, daß Rheumafaktoren die IgG-Antikörper quasi präsentieren, so daß in einem Immunoassay für spezifische IgM-Antikörper die Rheumafaktoren gebunden werden. Wenn die Rheumafaktoren IgG-Moleküle mit der nachzuweisenden Spezifität gebunden haben, kann es zu falsch positiven Meßergebnissen kommen.

Dieses Problem beim klassenspezifischen Nachweis antigenspezifischer Antikörper ist Gegenstand der DE 33 03 793. Dort wird ein Verfahren zum Nachweis eines antigenspezifischen Antikörpers einer bestimmten Ig-Klasse ("IgX") beschrieben, bei dem Rheumafaktoren durch die Zugabe von Anti-IgG-Antikörpern entstört werden. In dem Verfahren wird das spezifische Antigen, beispielsweise ein Virusantigen, auf einen festen Träger aufgebracht. Das festphasengebundene Virusantigen wird mit der Probe in Kontakt gebracht. Im nächsten Schritt wird die ungebundene Probe entfernt und der festphasengebundene Komplex aus Antigen-IgX mit einem Anti-IgX-Antikörper nachgewiesen. Um Störungen durch Rheumafaktoren insbesondere bei IgM-Nachweisen zu vermeiden, wird die Probe vor dem Test mit Anti-IgG-Antikörper behandelt. Die so komplexierten IgG-Antikörper sind somit für einen Angriff durch Rheumafaktoren nicht mehr zugängig, so daß Rheumafaktoren keine antigenspezifischen IgG-Moleküle binden können, die wiederum falsch positive Ergebnisse hervorrufen könnten. Es werden jedoch alle IgG-Antikörper unabhängig von ihrer Spezifität gebunden. Das Ausfällen von störenden IgG-Antikörpern mit Anti-IgG-Antikörpern kann zu unerwünschten Präzipitaten und Eintrübungen führen, die den gesamten Test negativ beeinflussen können. Außerdem ist die Probenvorbehandlung durch Anti-IgG-Antikörper aufwendig.

Eine weitere Möglichkeit, die Störeinflüsse von Antikörpern anderer Klassen gleicher Spezifität auszuschalten, wird in der WO 96/14337 offenbart. Hierbei werden zur Entstörung von IgG-Antikörpern Antikörper oder Antikörperfragmente eingesetzt, die spezifisch mit dem Fd-Abschnitt der schweren Kette von IgG reagieren. Dadurch wird die Antigenbindungsfähigkeit der IgGs so stark maskiert, daß sie die spezifischen Antigene nicht mehr erkennen können. Ein ähnliches Konzept wird in der WO 96/14338 beschrieben. Dort werden Anti-Fd-Antikörper bzw. -fragmente als Entstörungsreagenz zur Entstörung von Rheumafaktoren eingesetzt. Die Entstörung durch ein hochspezifisches Anti-Fd-Reagenz ist dennoch aufwendig und kostspielig.

Mit den im Stand der Technik bekannten Verfahren ist es nicht möglich, in einem Einschrittverfahren einen antigenspezifischen Antikörper der Immunglobulinklasse IgM nachzuweisen, ohne daß aufwendige und teure Entstörreagenzien und/oder mehrere spezifische Antikörper hinzugefügt werden müssen. Die aus dem Stand der Technik bekannten immunologischen Nachweisverfahren nach dem Brückentestkonzept, bei denen ein markiertes Antigen und ein an eine Festphase bindefähiges Antigen verwendet wird, ermöglichen zwar einen einstufigen Test. Bisher konnten jedoch mit diesem einfachen Prinzip Antikörper der Klassen IgG und IgM nur gemeinsam nachgewiesen werden können.

Aufgabe war es daher, eine verbesserte Methode zum Nachweis von gegen ein spezifisches Antigen gerichteten Antikörpern der IgM-Klasse zur Verfügung zu stellen. Diese Methode sollte auf aufwendige und teure Entstörungsreagenzien verzichten und bevorzugt auf einem einstufigen Testprinzip bestehen, um vorteilhaft in automatisierten Systemen angewendet werden zu können.

Diese Aufgabe wird gelöst mit dem erfindungsgemäßen Verfahren zur Bestimmung eines antigenspezifischen Antikörpers der Immunglobulinklasse M durch Inkubation der Probe mit mindestens zwei verschiedenen Rezeptoren R₁ und R₂, wobei beide Rezeptoren mit dem Antikörper spezifisch bindefähig sind, R₁ an eine feste Phase gebunden ist oder gebunden werden kann und R₂ eine Markierung trägt, das dadurch gekennzeichnet ist, daß ein wesentlicher Bestandteil von R₁ ein vom zu bestimmenden Antikörper spezifisch erkannter Bindepartner in polymerer Form ist, und die in der Probe vorhandenen IgG-Moleküle gleicher Spezifität durch die Zugabe von Bindepartnern in monomerer Form entstört werden.

Die in der Probe vorhandenen IgA-, IgD- und IgE-Antikörper, die die gleiche Spezifität wie die nachzuweisenden IgM-Antikörper besitzen, kommen im Vergleich zu IgG-Antikörpern in sehr niedrigen Konzentrationen vor, so daß kein Störeffekt durch die Klassen IgA, IgD und IgE zu erwarten ist. Bei den Antikörperklassen IgA, IgD und IgE handelt es sich wie bei IgG-Molekülen - und im Gegensatz zu den als Pentamer vorliegenden IgM-Antikörpern - um Antikörper, die in Form von einzelnen Molekülen vorliegen und jeweils zwei Bindungsstellen für das Antigen besitzen. Aufgrund der strukturellen Ähnlichkeit von IgG-, IgA-, IgD- und IgE-Antikörpern ist es daher zu vermuten, daß durch das im folgenden beschriebene erfindungsgemäße Verfahren zum antigenspezifischen IgM-Nachweis neben IgG-Antikörpern auch IgD-, IgA- und IgE-Antikörper entstört werden.

Das erfindungsgemäße Verfahren erlaubt die Bestimmung von antigenspezifischen Antikörpern der Immunglobulinklasse M in Proben, in denen Antikörper der Klasse IgG gleicher Antigenspezifität vorhanden sind. Außerdem kann das erfindungsgemäße Verfahren in Gegenwart von Rheumafaktoren durchgeführt werden. Eine aufwendige Vorbehandlung der Probe ist nicht notwendig.

Es hat sich überraschenderweise gezeigt, daß es durch den erfindungsgemäßen Einsatz von Bindepartnern in monomerer Form in einem Immunoassay zum Nachweis von antigenspezifischen IgM-Antikörpern möglich ist, IgG-Antikörper mit der gleichen Antigenspezifität effektiv zu entstören. Die Bindepartner in monomerer Form binden dabei spezifisch an die Antigenbindungsstellen der IgG-Antikörper. Die nachzuweisenden IgM-Antikörper mit gleicher Spezifität, die in der gleichen Probe vorhanden sind, reagieren überraschenderweise nicht oder lediglich vernachlässigbar schwach mit den Bindepartnern in monomerer Form. Der Begriff "vernachlässigbar schwach" bedeutet, daß die Antigenbindungsstellen der IgM-Antikörper nicht durch die Bindepartner in monomerer Form blockiert werden. Dies liegt vermutlich an der wesentlich geringeren Affinität der pentameren IgM-Antikörper zu monomer vorliegenden Epitopen im Vergleich zu den in Form einzelner Moleküle vorliegenden IgG-Antikörpern, die eine wesentlich höhere Affinität zu monomer vorliegenden Epitopen haben. Das heißt, daß trotz der Anwesenheit der Bindepartner in monomerer Form die Sensitivität des IgM-Nachweises nicht leidet. Die durch die Bindepartner in monomerer Form maskierten IgG-Antikörper stören den IgM-Nachweis nicht.

Es hat sich außerdem überraschenderweise gezeigt, daß auch Rheumafaktoren, die IgG-Antikörper gebunden haben, ebenfalls durch die Bindepartner in monomerer Form, die an die Antigenbindungsstellen der IgGs binden, effektiv entstört werden. Dadurch daß die Antigenbindungsstellen der IgG-Antikörper blockiert sind, kann der Nachweis der antigenspezifischen IgM-Antikörper ohne vorherige Abtrennung der IgG-Antikörper bzw. der Rheumafaktoren erfolgen. Die eingesetzten Bindepartner in monomerer Form sind nicht in der Lage, eine Agglutinationsreaktion der maskierten IgG-Antikörper bzw. der Rheumafaktoren auszulösen. Somit werden unerwünschte Eintrübungen durch Präzipitate verhindert, die die gesamte Testführung negativ beeinträchtigen können.

Aus diesem Grund ist bei den erfindungsgemäßen Verfahren eine sukzessive Testführung zur Abtrennung der IgG-Antikörper nicht unbedingt notwendig, da diese nicht stören. Ein besonderer Vorteil des Verfahrens ist daher die Einfachheit des Testablaufs.

Neben den sogenannten Naßtesten, bei denen die Testreagenzien in flüssiger Phase vorliegen, können auch alle gängigen Trockentestformate, die zum Nachweis von Proteinen bzw. Antikörpern geeignet sind, verwendet werden. Bei diesen Trockentests oder Teststreifen, wie sie beispielsweise in der EP-A-0 186 799 beschrieben sind, sind die Testkomponenten auf einem Träger aufgebracht. Wird das erfindungsgemäße Verfahren im Teststreifenformat durchgeführt, ist daher kein Wasch-Schritt notwendig. Bevorzugt wird das erfindungsgemäße Verfahren jedoch als Naßtest durchgeführt.

Es ist möglich, alle Rezeptoren und die Bindepartner in monomerer Form mit der Probe zusammen zu inkubieren und das Verfahren einstufig durchzuführen. Dies erfordert gegebenenfalls nur einen einzigen Wasch-Schritt nach der Inkubation.

Zur Durchführung des erfindungsgemäßen Verfahrens werden im Normalfall zwei verschiedene Rezeptoren R₁ und R₂ und die Bindepartner in monomerer Form verwendet. Wird ein Naßtest verwendet, so liegt der Rezeptor R₂ in flüssiger Phase vor. R₁ kann in flüssiger Phase oder bereits an die Festphase gebunden vorliegen. Die Bindepartner in monomerer Form liegen bevorzugt in flüssiger Phase vor.

Wird als R₁ ein mit einer Festphase bindefähiger Rezeptor eingesetzt, der noch nicht an der Festphase gebunden ist, so wird die Probe mit den Rezeptoren R₁ und R₂ und den Bindepartnern in monomerer Form gemeinsam inkubiert. Der Probenantikörper bindet dabei an R₁ und R₂. Diese Inkubation kann in Anwesenheit der Festphase stattfinden. Es bildet sich dabei ein Komplex aus Festphase-R₁-Probenantikörper-R₂. Anschließend wird die feste von der flüssigen Phase getrennt, gegebenenfalls die feste Phase gewaschen und die Markierung von R₂ gemessen. Die Markierung wird zumeist in der festen Phase gemessen, sie kann jedoch auch in der flüssigen Phase bestimmt werden.

Wird die Inkubation der Probe mit R₁ und R₂ und den Bindepartnern in monomerer Form in Abwesenheit der Festphase durchgeführt, so muß der gesamte Testansatz anschließend mit der Festphase in Kontakt gebracht, gegebenenfalls eine Waschung durchgeführt und die Markierung gemessen werden,

Liegt der Rezeptor R₁ bereits in festphasengebundener Form vor, so werden die Probe und der Rezeptor R₂ zum festphasengebundenen Rezeptor R₁ dazugegeben und zusammen inkubiert. Bevorzugt wird die Probe bei dieser Testführung mit den Bindepartnern in monomerer Form und R₂ vorinkubiert, bevor der Testansatz zum festphasengebundenen Rezeptor R₁ gegeben wird. Das weitere Vorgehen entspricht dem oben angegebenen Verfahren.

Es ist auch möglich, das erfindungsgemäße Verfahren in mehreren Stufen durchzuführen. In diesem Fall wird bevorzugt die Probe zunächst mit den Bindepartnern in monomerer Form und danach mit den Rezeptoren R₁ und R₂ inkubiert. Der Testansatz kann anschließend mit weiteren Rezeptoren inkubiert werden, wobei dies in mehreren Stufen erfolgen kann. Der weitere Testablauf entspricht dem vorher beschriebenen Verfahren.

Ein wesentlicher Bestandteil von R₁ ist ein vom zu bestimmenden IgM-Antikörper spezifisch erkannter Bindepartner in polymerer Form, der auch als Polyhapten bezeichnet werden kann. Unter einem erfindungsgemäßen Bindepartner in polymerer Form werden Strukturen verstanden, bei denen mehrere, vorzugsweise gleiche oder gleichartige, äquivalente Epitopbereiche an einen Träger gekoppelt sind, die eine spezifische Reaktion mit dem zu bestimmenden Antikörper zeigen. Der Begriff "gleichartig" oder "äquivalent" bedeutet, daß die auf dem Bindepartner in polymerer Form vorhandenen Strukturen nicht unbedingt alle identisch sein müssen. Die einzige Bedingung ist, daß die zu bestimmenden IgM-Antikörper spezifisch an diese Epitopbereiche binden. Der Epitopbereich kann beispielsweise aus einem Antigen oder einem Antiidiotyp-Antikörper abgeleitet sein. Der Epitopbereich kann auch aus Zuckerketten, wie sie beispielsweise in glykierten Proteinen vorkommen, abgeleitet sein.

Er kann weiterhin von Lipid-Strukturen abgeleitet sein, wie sie zum Beispiel in Phospholipiden oder Lipoproteinen vorkommen. Das Polyhapten bzw. der Bindepartner in polymerer Form besteht somit aus vielen gleichen oder gleichartigen Epitopbereichen, weist also, wie bereits weiter oben angeführt, viele gleichartige Bindungsstellen für den Probenantikörper auf. Unter einer Bindungsstelle ist im Falle eines Proteins als Antigen ein Peptid zu verstehen, dessen Sequenz ein Teil der Proteinsequenz eines Proteinantigens (Analyt) ist und an das ein gegen dieses Protein gerichteter Antikörper spezifisch bindet. Im Falle eines Antigens, das eine Zuckerstruktur umfaßt, wäre die Bindungsstelle der Bereich aus Zuckermolekülen, an den der Probenantikörper spezifisch bindet. Bei Lipid-Strukturen können die Lipidmoleküle die Bindungsstelle für den Probenantikörper darstellen. Eine Bindungsstelle kann auch aus Kombinationen von peptidischen Bereichen mit Zuckern und/oder Lipiden bestehen. Bevorzugt werden als Bindepartner in polymerer Form jedoch Polyhaptene auf Peptidbasis eingesetzt. Bei den erfindungsgemäßen Bindepartnern in polymerer Form ist vor allem eine hohe Epitopdichte wichtig, damit die pentameren IgM-Probenantikörper spezifisch mit hoher Affinität an den Bindepartner in polymerer Form binden können. Die weiteren Bedingungen für die einzelnen Peptidbestandteile der erfindungsgemäßen Bindepartner in polymerer Form entsprechen den weiter unten für die Bindepartner in monomerer Form aufgeführten Voraussetzungen.

Als Trägermaterial der Polyhaptene können Teilchen aus beispielsweise Latex, Polystyrol, Polyacrylat, Polymethacrylat oder Gold verwendet werden. Auch Polymere wie Dextran, oder Polypeptide wie beispielsweise Polylysin, Rinderserum-Albumin, ß-Glactosidase, unspezifische Immunglobuline oder deren Fragmente können als Trägermaterial der Polyhaptene verwendet werden. Die einzige Bedingung bei der Wahl des Trägers ist, daß er keine Kreuzreaktivität mit Antikörpern in der Probenflüssigkeit zeigt. Eine weitere Bedingung ist, daß sich die Haptene an den Träger koppeln lassen müssen. Die Epitopbereiche oder Haptene werden nach dem Fachmann bekannten Methoden wie beispielsweise in der EP-A-0 650 053 und der WO 96/03652 beschrieben an das Trägermaterial gekoppelt. Zwischen Epitopbereich und Trägermaterial können außerdem Spacerbereiche eingefügt werden, die ebenfalls in den zuvor genannten Offenlegungsschriften offenbart sind. Es können alle dem Fachmann bekannten Spacerbereiche verwendet werden. Voraussetzung ist, daß sie immunologisch inaktiv sind, das heißt, daß sie nicht mit Antikörpern in der Probe kreuzreagieren.

R₁ kann entweder direkt an die Festphase gebunden sein, oder die Bindung an die Festphase erfolgt indirekt über ein spezifisches Bindungssystem. Die direkte Bindung von R₁ an die Festphase erfolgt nach dem Fachmann bekannten Methoden. Wird die Bindung indirekt über ein spezifisches Bindungssystem durchgeführt, so ist R₁ ein Konjugat, das aus einem erfindungsgemäßen Bindepartner in polymerer Form und einem Reaktionspartner eines spezifischen Bindungssystems besteht. Unter einem spezifischen Bindungssystem werden hier zwei Partner verstanden, die spezifisch miteinander reagieren können. Das Bindungsvermögen kann dabei auf einer immunologischen Reaktion oder auf einer anderen spezifischen Reaktion beruhen. Bevorzugt wird als spezifisches Bindungssystem eine Kombination von Biotin und Avidin oder Biotin und Streptavidin verwendet. Weitere bevorzugte Kombinationen sind Biotin und Antibiotin, Hapten und Anti-Hapten, Fc-Fragment eines Antikörpers und Antikörper gegen dieses Fc-Fragment oder Kohlenhydrat und Lectin. Einer der Reaktionspartner dieses spezifisch bindefähigen Paares ist dann Teil des Konjugates, das den Rezeptor R₁ bildet.

Der andere Reaktionspartner des spezifischen Bindungssystems liegt in fester Phase vor.
Die Bindung des anderen Reaktionspartners des spezifischen Bindungssystems an ein unlösliches Trägermaterial kann nach den üblichen, dem Fachmann bekannten Methoden vorgenommen werden. Hierbei ist sowohl eine kovalente als auch eine adsorptive Bindung geeignet. Als feste Phase besonders geeignet sind Reagenzgläschen oder Mikrotiterplatten aus Polystyrol oder ähnlichen Kunststoffen, die an der Innenoberfläche mit Reaktionspartner des spezifischen Bindungssystems beschichtet sind. Weiterhin geeignet und besonders bevorzugt sind teilchenförmige Substanzen, wie beispielsweise Latexpartikel, Molekularsiebmaterialien, Glaskörperchen, Kunststoffschläuche und dergleichen. Auch poröse, schichtförmige Träger wie Papier können als Träger verwendet werden.

Der Rezeptor R₂ besteht aus einem Molekül, das spezifisch mit dem Probenantikörper reagiert und einer Markierung. Das spezifisch mit dem Probenantikörper reagierende Molekül kann beispielsweise entweder ein spezifisch an den Probenantikörper bindender Antikörper, ein Antikörperfragment, ein Protein, ein Antigen, ein Hapten sein. Die einzige Bedingung für das Molekül als Bestandteil von R₂ ist, daß es spezifisch mit dem nachzuweisenden Probenantikörper reagiert. Bevorzugt ist dieses Molekül ein erfindungsgemäßer Bindepartner in polymerer Form, das den Probenantikörper spezifisch bindet. Die in R₂ enthaltenen Bindepartner in polymerer Form werden nach den gleichen Methoden hergestellt wie die Bindepartner in polymerer Form für R₁.

Ein weiterer Bestandteil des Rezeptors R₂ ist die Markierung. Bevorzugt wird als Markierung eine direkt nachweisbare Substanz, beispielsweise eine chemilumineszierende, fluoreszierende oder radioaktive Substanz oder ein Metallsol-, Latex- oder Goldpartikel eingesetzt. Weiterhin bevorzugt als Markierung sind Enzyme oder andere biologische Moleküle wie beispielsweise Haptene. Unter den Haptenen ist Digoxigenin eine besonders bevorzugte Markierung. Die Verfahren zur Markierung sind dem Fachmann geläufig und bedürfen hier keiner weiteren Erklärung. Der Nachweis der Markierung erfolgt in an sich bekannter Weise direkt durch Messung der chemilumineszierenden, fluoreszierenden oder radioaktiven Substanz oder des Metallsol-, Latex- oder Goldpartikels oder durch Messung des durch das Enzym umgesetzten Substrats.

Der Nachweis der Markierung kann auch auf indirekte Weise erfolgen. Dabei bindet ein weiterer Rezeptor, der selbst wiederum mit einer signalerzeugenden Gruppe gekoppelt ist, spezifisch an die Markierung von R₂, beispielsweise ein Hapten wie Digoxigenin. Der Nachweis der signalerzeugenden Gruppe, beispielsweise eine chemilumineszierende, fluoreszierende oder radioaktive Substanz oder ein Enzym oder Goldpartikel, erfolgt nach dem Fachmann geläufigen Methoden. Als weiterer Rezeptor kann beispielsweise ein Antikörper oder ein Antikörperfragment eingesetzt werden, der spezifisch an die Markierung von R₂ bindet. Wird dieser indirekte Nachweis der Markierung verwendet, so ist die Markierung von R₂ bevorzugt Digoxigenin oder ein anderes Hapten, und der Nachweis erfolgt über einen mit Peroxidase gekoppelten Antikörper, der gegen Digoxigenin oder gegen das Hapten gerichtet ist.

Zur Entstörung von IgG-Antikörpern gleicher Antigenspezifität werden erfindungsgemäß Bindepartner in monomerer Form zum Testansatz gegeben. Der Begriff "monomer" bedeutet, daß die erfindungsgemäßen Bindepartner in monomerer Form nur einen Epitopbereich bzw. nur eine Bindungsstelle für den zu entstörenden Antikörper enthalten, d.h. eine immunologisch spezifisch mit dem IgG-Antikörper reagierende Struktur. Die monomere Struktur dieser Bindepartner ist wichtig, um zu gewährleisten, daß nur die zu entstörenden antigenspezifischen IgG-Antikörper an die Bindepartner in monomerer Form binden und nicht die nachzuweisenden IgM-Antikörper.

Der Epitopbereich kann - wie bei den weiter oben beschriebenen Bindepartnern in polymerer Form - beispielsweise aus einem Antigen oder einem Antiidiotyp-Antikörper abgeleitet sein. Entsprechend den Bedingungen für die Bindepartner in polymerer Form können die Epitopbereiche auch bei den Bindepartnern in monomerer Form von Zucker- und/oder Lipid-Strukturen oder kombinierten Strukturen mit peptidischem, lipidischen und/oder Zuckeranteil abgeleitet sein. Es können alle von einem Epitopbereich abgeleiteten Strukturen verwendet werden, die eine Bindungsstelle aufweisen, an die der zu entstörende Antikörper der Klasse IgG in Anwesenheit von IgM-Antikörpern gleicher Spezifität spezifisch bindet. Die einzige Bedingung für die Bindungstelle, d.h. für den eingesetzten Bindepartner in monomerer Form ist, daß die spezifische Bindefähigkeit zu IgG erhalten bleibt. Diese Bedingung gilt auch für den Fall, daß Zucker- oder Lipidstrukturen in der Bindungstelle enthalten sind.

Erfindungsgemäß können auch Bindepartner in monomerer Form eingesetzt werden, die die Bindungsstelle, an die der zu entstörende IgG-Antikörper spezifisch bindet, flankieren oder überlappen. Somit ist es möglich, auch IgG-Antikörper zu entstören, deren Bindungsstelle ein Epitop umfaßt, das nicht exakt identisch mit dem Epitop ist, das von den nachzuweisenden IgM-Antikörpern erfaßt wird. Diese IgG-Antikörper können eine mehr oder weniger stark ausgeprägte Kreuzreaktivität mit den nachzuweisenden IgM-Antikörpern aufweisen. Durch die Zugabe von Bindepartnern in monomerer Form, die den Epitopen dieser kreuzreagierenden Antikörper entsprechen und damit eine hohe Affinität zu diesen besitzen, werden auch diese IgG-Antikörper entstört. Bevorzugt wird zur Entstörung der IgG-Antikörper ein Gemisch aus Bindepartnern in monomerer Form eingesetzt, die mit den Epitopen der nachzuweisenden IgM-Antikörper mehr oder weniger stark überlappen.

Bevorzugt werden Peptide als Bindepartner in monomerer Form eingesetzt. Unter einer Bindungsstelle ist - analog zur Definition des Bindepartners in polymerer Form - im Falle eines Proteins als Analyt ein Peptid zu verstehen, dessen Sequenz ein Teil der Proteinsequenz eines Proteinantigens ist und an das ein gegen dieses Protein gerichteter Antikörper, der in der vorliegenden Erfindung ein IgG-Antikörper ist, spezifisch bindet. Neben diesen Peptiden sind unter einer Bindungsstelle auch noch Peptide mit Aminosäuresequenzen zu verstehen, die eine im wesentlichen äquivalente Spezifität und/oder Affinität der Bindung an den zu entstörenden IgG-Antikörper wie die zuvor genannten Peptide zeigen. Diese Peptide können vorzugsweise durch Substitution, Deletion oder Insertion einzelner Aminosäurereste aus den zuvor genannten Peptiden abgeleitet werden.

Unter den erfindungsgemäßen Peptiden, die einer spezifischen Bindungsstelle entsprechen, sind auch Peptidderivate zu verstehen, in denen eine oder mehrere Aminosäuren durch eine chemische Reaktion derivatisiert worden sind. Beispiele von erfindungsgemäßen Peptidderivaten sind insbesondere solche Moleküle, in denen das Backbone oder/und reaktive Aminosäureseitengruppen, zum Beispiel freie Aminogruppen, freie Carboxylgruppen oder/und freie Hydroxylgruppen, derivatisiert worden sind. Spezifische Beispiele für Derivate von Aminogruppen sind Sulfonsäure- oder Carbonsäureamide, Thiourethanderivate und Ammoniumsalze, zum Beispiel Hydrochloride. Carboxylgruppenderivate sind Salze, Ester und Amide. Beispiele für Hydroxylgruppenderivate sind O-Acyl- oder O-Alkylderivate. Die Herstellung der Peptide erfolgt bevorzugt durch chemische Synthese nach dem Fachmann bekannten Methoden und bedürfen hier keiner besonderen Erläuterung.

Weiterhin umfaßt der Begriff Peptidderivat auch solche Peptide, in denen eine oder mehrere Aminosäuren durch natürlich vorkommende oder nicht-natürlich vorkommende Aminosäurehomologe der 20 "Standard"-Aminosäuren ersetzt werden. Beispiele für solche Homologe sind 4-Hydroxyprolin, 5-Hydroxylysin, 3-Methylhistidin, Homoserin, Ornithin, β-Alanin und 4-Aminobuttersäure. Die Peptidderivate müssen eine im wesentlichen äquivalente Spezifität oder/und Affinität der Bindung an die zu entstörenden IgG-Antikörper aufweisen, wie die Peptide, aus denen sie abgeleitet sind.

Als erfindungsgemäße Peptide, die einer spezifischen Bindungsstelle entsprechen, werden auch peptidmimetische Substanzen, im folgenden Peptidmimetika genannt, zu verstehen, die eine im wesentlichen äquivalente Spezifität oder/und Affinität der Bindung an die zu entstörenden IgG-Antikörper aufweisen, wie die zuvor genannten Peptide oder Peptidderivate. Peptidmimetika sind Verbindungen, die Peptide in ihrer Wechselwirkung mit dem zu bestimmenden Antikörper ersetzen können und gegenüber den nativen Peptiden eine erhöhte Stabilität, insbesondere gegenüber Proteinasen oder Peptidasen, aufweisen können. Methoden zur Herstellung von Peptidmimetika sind beschrieben bei Giannis und Kolter, Angew. Chem. 105 (1993), 1303-1326 und Lee et al., Bull.Chem. Soc. Jpn. 66 (1993), 2006-2010.

Die Länge einer Bindungsstelle, d.h. die Länge des erfindungsgemäßen monomeren Peptids beträgt üblicherweise mindestens 4 Aminosäuren. Bevorzugt liegt die Länge zwischen 4 bis 20, 6 bis 15 oder besonders bevorzugt bei 9 bis 12 Aminosäuren. Im Falle der Peptidmimetika oder Peptidderivate ist eine analoge Länge beziehungsweise Größe des Moleküls notwendig.

Die erfindungsgemäßen monomeren Peptide als Bindepartner in monomerer Form enthalten das Epitop, an das der zu entstörende IgG-Antikörper spezifisch bindet. Am N-terminalen und/oder am C-terminalen Ende des Peptids können jedoch noch weitere flankierende Peptidsequenzen vorhanden sein, die nicht mehr dem spezifischen Epitop entsprechen. Diese Maßnahme kann unter Umständen notwendig sein, um die Löslichkeit des Peptids zu verbessern. Die einzigen Bedingungen sind, daß das Peptid als Bindepartner in monomerer Form tatsächlich monomer vorliegt und die starke Bindefähigkeit an die zu entstörenden IgG-Antikörper erhalten bleibt.

Voraussetzung beim Einsatz der Bindepartner in monomerer Form (hier: Peptide) ist, daß sie das gleiche Epitop in monomerer Form enthalten wie dasjenige Epitop, das auf den Bindepartnern in polymerer Form bzw. Polyhaptenen in polymerer Form vorhanden ist. Dies bedeutet, daß für eine effektive Entstörung die IgG-Antikörper, die an die Bindepartner in monomerer Form binden, immunologisch die gleiche Spezifität besitzen müssen wie die nachzuweisenden IgM-Antikörper. Entstört werden sollen beim Nachweis der IgM-Antikörper immer IgG-Antikörper gleicher Antigenspezifität.

Bevorzugt werden die Bindepartner in monomerer Form in einem 10- bis 10000-fachen Überschuß im Vergleich zur Konzentration der Epitope auf den Bindepartnern in polymerer Form eingesetzt. Bevorzugt werden die Bindepartner in monomerer Form in 10- bis 1000- und besonders bevorzugt in 100-fachem Überschuß eingesetzt. Eine Obergrenze für die Konzentration an Bindepartnern in monomerer Form gibt es nur insofern, als ab einer bestimmten Konzentration die Löslichkeit der Bindepartner in monomerer Form nicht mehr gegeben ist. Die Konzentration der Epitope auf den Bindepartnern in polymerer Form ist abhängig von der Größe des Trägermaterials und kann vom Fachmann leicht für jede Testführung und jeden Nachweisparameter individuell ermittelt werden. Als Richtwert für die Konzentration der Epitope auf den Bindepartnern in polymerer Form hat sich in Falle von peptidischen Epitopen (Länge: 6 bis 20 Aminosäuren) eine Konzentration von 5 bis 500 ng Peptid pro ml Trägermaterial als geeignet erwiesen.

Neben den Bindepartnern in monomerer Form können außerdem zusätzliche Entstörmittel wie beispielsweise die im einleitenden Teil beschriebenen Anti-Fd-Antikörper eingesetzt werden, die einen zusätzlichen Entstöreffekt für IgG-Antikörper bewirken.

Als Testführungen für den antigenspezifischen IgM-Nachweis kommen sowohl heterogene als auch homogene Verfahren in Frage. Bei einer homogenen Testführung erfolgt die Bindung des Komplexes aus R₁, Proben-IgM und R₂ nicht an eine Festphase. Vielmehr entsteht durch die Agglutination mehrerer solcher Komplexe miteinander eine Eintrübung, die ein Maß für die Konzentration an IgM darstellt. Die als IgG-Entstörer wirkenden Bindepartner in monomerer Form hemmen die Agglutination nicht.

Bevorzugt wird jedoch eine heterogenes Verfahren durchgeführt. Besonders bevorzugt wird das Verfahren nach dem Brückentest-Prinzip (siehe EP-A-0 280 211) durchgeführt. In einer der bevorzugten Ausführungsformen wird als R₁ ein Konjugat aus einem erfindungsgemäßen Polyhapten und einem Bindungspartner eines spezifischen Bindungssystems, bevorzugt Biotin, eingesetzt. R₂ besteht aus einem erfindungsgemäßen Polyhapten bzw.

Bindepartner in polymerer Form und einer Markierung, bevorzugt Digoxigenin. Besonders bevorzugt werden in R₁ und R₂ die gleichen Polyhaptene verwendet. Die Rezeptoren R₁ und R₂, werden in dieser bevorzugten Ausführung in Anwesenheit der in diesem Fall bevorzugt mit Avidin oder Streptavidin beschichteten Festphase gleichzeitig mit der Probe und den Bindepartnern in monomerer Form, bevorzugt Peptiden, inkubiert. Dabei reagieren die Bindepartner in polymerer Form von R₁ und R₂ spezifisch mit den zu bestimmenden IgM-Antikörpern, während die IgG-Antikörper gleicher Spezifität durch die Peptide, d.h. die Bindepartner in monomerer Form maskiert werden, so daß sie nicht an die Bindepartner in polymerer Form binden können. Somit ist der gesamte Komplex aus Avidin/Streptavidin-R₁-IgM-Probenantikörper-R₂ an die Festphase gebunden. Nach Trennung der Festphase von der flüssigen Phase und gegebenenfalls Waschung der festen Phase wird der festphasengebundene Komplex mit einem weiteren Rezeptor (hier mit einem gegen Digoxigenin gerichteten Antikörper) inkubiert, der spezifisch die Markierung von R₂ erkennt. Der weitere Rezeptor ist mit einer signalerzeugenden Gruppe, bevorzugt mit dem Enzym Peroxidase gekoppelt. Nach gegebenenfalls einem weiteren Wasch-Schritt erfolgt der Nachweis des Probenantikörpers über die signalerzeugende Gruppe, in diesem Fall durch das vom Enzym umgesetzte Substrat. Bei dieser Testführung kann die Inkubation der Probe mit R₁, R₂, den Bindepartnern in monomerer Form und dem weiteren Rezeptor auch gleichzeitig erfolgen. Dadurch vereinfacht sich die Testführung nochmals.

Die oben beschriebene Testführung ist gut für den Einsatz in automatisierten Systemen geeignet. Es können auch mehrere antigenspezifische IgM-Antikörper nachgewiesen werden wie beispielsweise HIV-Antikörper gegen verschiedene HIV-Antigene. In einem solchen Fall kann der weitere Rezeptor als Universalmarkierung eingesetzt werden, da der weitere Rezeptor spezifisch die Markierung von R₂ erkennt. Werden mehrere IgM-Antikörper mit unterschiedlicher Antigenspezifität gleichzeitig nachgewiesen, müssen die Polyhapten-Bestandteile von R₁ und R₂ sowie die zur Entstörung eingesetzten Bindepartner in monomerer Form die entsprechenden Spezifitäten besitzen.

Als Proben können alle dem Fachmann bekannten biologischen Flüssigkeiten verwendet werden. Bevorzugt werden als Probe Körperflüssigkeiten wie Vollblut, Blutserum, Blutplasma, Urin, Speichel etc. eingesetzt.

In den Testansätzen können neben der Probe, der Festphase und den aufgeführten Rezeptoren weitere, je nach Anwendung benötigte Zusätze wie Puffer, Salze, Detergenzien, Proteinzusätze wie beispielsweise RSA vorhanden sein. Die notwendigen Zusätze sind dem Fachmann bekannt oder können von ihm auf einfache Weise herausgefunden werden.

Ein weiterer Gegenstand der Erfindung ist ein Reagenz zur Bestimmung eines antigenspezifischen Antikörpers der Immunglobulinklasse M, das neben den üblichen Testzusätzen für Immunoassays Bindepartner in monomerer Form, d.h. bevorzugt Peptide, und einen mit dem zu bestimmenden Antikörper bindefähigen Rezeptor R₁ enthält, der mit einer Festphase bindefähig ist und dessen wesentlicher Bestandteil ein vom zu bestimmenden Antikörper spezifisch erkannter Bindepartner in polymerer Form ist.

Ebenfalls ein Gegenstand der Erfindung ist ein Reagenz zur Bestimmung eines antigenspezifischen Antikörpers der Immunglobulinklasse M, das neben den üblichen Testzusätzen für Immunoassays Bindepartner in monomerer Form, d.h. bevorzugt Peptide und zwei mit dem zu bestimmenden Antikörper bindefähige Rezeptoren R₁ und R₂ enthält, von denen R₁ mit einer Festphase bindefähig ist und R₂ eine Markierung trägt, wobei ein wesentlicher Bestandteil der Rezeptoren R₁ und R₂ jeweils ein vom zu bestimmenden Antikörper spezifisch erkannter Bindepartner in polymerer Form ist.

Ein weiterer Gegenstand der Erfindung ist die Verwendung von Bindepartnern in polymerer Form zur antigenspezifischen Bestimmung von IgM-Antikörpern nach einem der vorhergehend genannten erfindungsgemäßen Verfahren.

Des weiteren ist ein Gegenstand der vorliegenden Erfindung die Verwendung von Bindepartnem in monomerer From, d.h. bevorzugt Peptide zur Entstörung von IgG-Antikörpern und/oder Rheumafaktoren bei der Bestimmung von antigenspezifischen IgM-Antikörpern.

Das folgende Beispiel erläutert die Erfindung weiter.

### Beispiel

### Antigenspezifischer IgM-Test: anti-HIV 2-IgM

Biotin-markierte und Digoxigenin-markierte multimere Antigene (HIV 2) werden mit Probenantikörpern und Streptavidin-beschichteter Festphase inkubiert (Inkubation bei 25°C oder 37°C, ca. 60 bis 180 min, in diesem Beispiel: 120 min, 25°C). Nach einem Wasch-schritt reagiert der wandgebundene Immunkomplex mit anti-Digoxigenin-Peroxidase-Konjugat (Inkubation bei 25°C oder 37°C, ca. 30 bis 120 min, in diesem Beispiel: 60 min 25°C). Nach einem weiteren Waschschritt wird der Peroxidase-Konjugat-markierte Immunkomplex durch eine Substratreaktion nachgewiesen (Inkubation bei 25°C oder 37°C, ca. 30 bis 120 min, in diesem Beispiel: 60 min 25°C).

Die Reaktionsschritte (außer der Substrat-Reaktion) finden statt in einem Tris/HCl Puffer (pH 7.5, 50 bis 150 mM, in diesem Beispiel 100 mM) mit ca. 0.05 bis 0.4 % Detergenz (hier 0.2 % Polidocanol), und ca. 0.5 % Protein/Proteinderivat-Zusätze (hier u.a. Pepton aus Lactalbumin und RSA).

Die Probenantiköper sind in diesem Fall monoclonale Maus-Antiköper (IgM und IgG) gegen ein HIV 2-Epitop verdünnt auf ca. 2 - 20 µg/ml in anti-HIV negativem Humanserum.

Die Kompetition erfolgt mit freiem unmarkierten HIV 2-Peptidantigen in 10-fachem bzw. 100-fachem Überschuß im Vergleich zur Konzentration der Peptidepitope auf den Polyhaptenen.

| Testsignal in mE: | | | | | | |
|---|---|---|---|---|---|---|
| **Proben <HIV 2> MAKs** | **Polyhaptene ohne freies Peptid** | **Bewertung** | **Polyhaptene plus freies Peptid 10 facher Überschuß** | **Bewertung** | **Polyhaptene plus freies Peptid 100 facher Überschuß** | **Bewertung** |
| IgM 2.6.6. | 2460 | pos. | 2488 | pos. | 2457 | pos. |
| IgM 2.22.8 | 1950 | pos. | 1990 | pos. | 1985 | pos. |
| IgG A | 2490 | pos. | 376 | grenzw. | 189 | neg. |
| IgG B | 626 | pos. | 167 | neg. | 152 | neg. |
| IgG C | 5699 | pos. | 1575 | pos. | 286 | neg. |
| | | **Test erkennt IgM und IgG** | | **Test erkennt IgM und etwas IgG** | | **Test IgM- spezifisch** |

Durch den Zusatz von monomeren Peptiden erfolgt der HIV 2-Antikörpernachweis IgM-spezifisch. Ohne den Peptidzusatz liefern einige Proben falsch positive Testergebnisse.

## Patentansprüche

1. Verfahren zur Bestimmung eines antigenspezifischen Antikörpers der Immunglobulinklasse M durch Inkubation der Probe mit mindestens zwei verschiedenen Rezeptoren R₁ und R₂, wobei beide Rezeptoren mit dem Antikörper spezifisch bindefähig sind, R₁ an eine feste Phase gebunden ist oder gebunden werden kann und R₂ eine Markierung trägt, dadurch gekennzeichnet, daß ein wesentlicher Bestandteil von R₁ ein vom zu bestimmenden Antikörper spezifisch erkannter Bindepartner in polymerer Form ist, und die in der Probe vorhandenen IgG-Moleküle gleicher Spezifität durch Zugabe von Bindepartnern in monomerer Form entstört werden.

2. Verfahren nach Anspruch 1, dadurch gekennzeichnet, daß ein wesentlicher Bestandteil von R₁ und R₂ jeweils ein vom zu bestimmenden Antikörper spezifisch erkannter Bindepartner in polymerer Form ist, und die in der Probe vorhandenen IgG-Moleküle gleicher Spezifität durch Zugabe von Bindepartnern in monomerer Form entstört werden.

3. Verfahren nach Anspruch 1 oder 2, dadurch gekennzeichnet, daß als spezifisches Bindungssystem für die Bindung von R₁ an die Festphase Biotin/Avidin; Biotin/Streptavidin; Biotin/Antibiotin; Hapten/Antihapten; Fc-Fragment eines Antikörpers/Antikörper gegen dieses Fc-Fragment; Kohlenhydrat/Lectin verwendet wird.

4. Verfahren nach einem der Ansprüche 1 bis 3, dadurch gekennzeichnet, daß der Rezeptor R₂ mit einer chemilumineszierenden, fluoreszierenden oder radioaktiven Substanz, einem Enzym oder einem anderen biologischen Molekül markiert ist.

5. Verfahren nach einem der Ansprüche 1 bis 4, dadurch gekennzeichnet, daß man die Probe gleichzeitig mit R₁ und R₂ und den zur Entstörung eingesetzten Bindepartnern in monomerer Form inkubiert.

6. Verfahren nach einem der Ansprüche 1 bis 5, dadurch gekennzeichnet, daß der Testansatz mit einem weiteren Rezeptor, der spezifisch an die Markierung des Rezeptors R₂ bindet, inkubiert wird, wobei der weitere Rezeptor ein Konjugat aus einem für die Markierung von R₂ spezifischen Rezeptor und einer Markierung ist, und anschließend die Markierung bestimmt wird.

7. Verfahren nach einem der Ansprüche 1 bis 6, dadurch gekennzeichnet, daß die zur Entstörung verwendeten Bindepartner in monomerer Form in 10- bis 10000-fachem Überschuß im Vergleich zur Konzentration der Epitope auf den Bindepartnern in polymerer Form von R₁ und R₂ zugesetzt werden.

8. Reagenz zur Bestimmung eines antigenspezifischen Antikörpers der Immunglobulinklasse M nach einem Verfahren gemäß den vorhergehenden Ansprüchen, dadurch gekennzeichnet, daß es neben den üblichen Testzusätzen für Immunoassays Bindepartner in monomerer Form und einen mit dem zu bestimmenden Antikörper bindefähigen Rezeptor R₁ enthält, der mit einer Festphase bindefähig ist und dessen wesentlicher Bestandteil ein Bindepartner in polymerer Form ist.

9. Reagenz zur Bestimmung eines antigenspezifischen Antikörpers der Immunglobulinklasse M nach einem Verfahren gemäß den vorhergehenden Ansprüchen, dadurch gekennzeichnet, daß es neben den üblichen Testzusätzen für Immunoassays Bindepartner in monomerer Form und zwei mit dem zu bestimmenden Antikörper bindefähige Rezeptoren R₁ und R₂ enthält, deren wesentlicher Bestandteil jeweils ein Bindepartner in polymerer Form ist, wobei R₁ mit einer Festphase bindefähig ist und R₂ eine Markierung trägt.

10. Verwendung von Bindepartnern in monomerer Form zur Entstörung von IgG-Antikörpern und/oder Rheumafaktoren bei der Bestimmung eines antigenspezifischen IgM-Antikörpers.

## Claims

1. Method for the determination of an antigen-specific antibody of the immunoglobulin M class by incubating the sample with at least two different receptors R₁ and R₂ where both receptors are capable of binding specifically to the antibody, R₁ is bound or can be bound to a solid phase and R₂ carries a label, wherein an essential component of R₁ is a binding partner in a polymeric form which is specifically recognized by the antibody to be determined, and interference by IgG molecules of the same specificity present in the sample is reduced by adding binding partners in a monomeric form.

2. Method as claimed in claim 1, wherein an essential component of R₁ and R₂ is a binding partner in a polymeric form which is specifically recognized by the antibody to be determined and interference by IgG molecules of the same specificity that are present in the sample is reduced by adding binding partners in a monomeric form.

3. Method as claimed in claim 1 or 2, wherein biotin/avidin, biotin/streptavidin, biotin/antibiotin, hapten/antihapten, Fc fragment of an antibody/antibody against this Fc fragment or carbohydrate/lectin is used as a specific binding system to bind R₁ to the solid phase.

4. Method as claimed in one of the claims 1 to 3, wherein the receptor R₂ is labelled with a chemiluminescent, fluorescent or radioactive substance or with an enzyme or another biological molecule.

5. Method as claimed in one of the claims 1 to 4, wherein the sample is simultaneously incubated with R₁ and R₂ and with the binding partners in monomeric form used to reduce interference.

6. Method as claimed in one of the claims 1 to 5, wherein the test mixture is incubated with an additional receptor which specifically binds to the label of the receptor R₂ in which the additional receptor is a conjugate of a specific receptor for the label of R₂ and a label, and subsequently the label is determined.

7. Method as claimed in one of the claims 1 to 6, wherein the binding partners in a monomeric form used to reduce interference are added in a 10-fold to 10,000-fold excess compared to the concentration of the epitopes on the binding partners in a polymeric form of R₁ and R₂.

8. Reagent for the determination of an antigen-specific antibody of the immunoglobulin M class by a method according to one of the previous claims, wherein in addition to the common test additives for immunoassays, it contains binding partners in a monomeric form and a receptor R₁ capable of binding to the antibody to be determined which is capable of binding to a solid phase and whose essential component is a binding partner in a polymeric form.

9. Reagent for the determination of an antigen-specific antibody of the immunoglobulin M class by a method according to one of the previous claims, wherein in addition to the common test additives for immunoassays, it contains binding partners in a monomeric form and two receptors R₁ and R₂ capable of binding to the antibody to be determined, whose essential component is a binding partner in a polymeric form and wherein R₁ is capable of binding to a solid phase and R₂ carries a label.

10. Use of binding partners in a monomeric form to reduce interference by IgG antibodies and/or rheumatoid factors in the determination of an antigen-specific IgM antibody.

## Revendications

1. Procédé de détermination d'un anticorps de la classe des immunoglobulines M et spécifique à un antigène, par incubation de l'échantillon avec au moins deux récepteurs R₁ et R₂ différents, les deux récepteurs pouvant se lier spécifiquement à l'anticorps, R₁ étant lié ou pouvant être lié à une phase solide et R₂ portant un marqueur, caractérisé en ce qu'un composant essentiel de R₁ est un partenaire de liaison sous forme polymère reconnu spécifiquement par l'anticorps à déterminer, et les molécules d'IgG de même spécificité présentes dans l'échantillon sont rendues inactives par addition de partenaires de liaison sous forme monomère.

2. Procédé selon la revendication 1, caractérisé en ce qu'un composant essentiel de R₁ et de R₂ est chaque fois un partenaire de liaison sous forme polymère reconnu spécifiquement par l'anticorps à déterminer, et les molécules d'IgG de même spécificité présentes dans l'échantillon sont rendues inactives par addition de partenaires de liaison sous forme monomère.

3. Procédé selon la revendication 1 ou 2, caractérisé en ce que comme système de liaison spécifique pour la liaison de R₁ à la phase solide, on utilise biotine/avidine ; biotine/streptavidine ; biotinelantibiotine ; haptène/antihaptène ; fragment Fc d'un anticorpslanticorps dirigé contre ce fragment ; hydrate de carbone/lectine.

4. Procédé selon l'une des revendications 1 à 3, caractérisé en ce que le récepteur R₂ est marqué par une substance chimioluminescente, fluorescente ou radioactive, par une enzyme ou par une autre molécule biologique.

5. Procédé selon l'une des revendications 1 à 4, caractérisé en ce que l'on incube l'échantillon en même temps que R₁ et R₂ et que les partenaires de liaison sous forme monomère utilisés pour l'inactivation.

6. Procédé selon l'une des revendications 1 à 5, caractérisé en ce que l'on incube l'ensemble de test avec un autre récepteur qui se lie spécifiquement au marqueur du récepteur R₂, l'autre récepteur étant un conjugué constitué d'un récepteur spécifique du marqueur de R₂ et d'un marqueur, et on détermine ensuite le marqueur.

7. Procédé selon l'une des revendications 1 à 6, caractérisé en ce que les partenaires de liaison sous forme monomère utilisés pour la désactivation sont ajoutés en excès de 10 à 10 000 fois par rapport à la concentration des épitopes présents sur les partenaires de liaison de R₁ et R₂ sous forme polymère.

8. Réactif pour la détermination d'un anticorps de la classe des immunoglobulines M et spécifique à un antigène, par un procédé selon les revendications précédentes, caractérisé en ce qu'en plus des additifs de test habituels pour les déterminations immunologiques, il contient des partenaires de liaison sous forme monomère et un récepteur R₁ capable de se lier à l'anticorps à déterminer, capable de se lier à une phase solide et dont le composant essentiel est un partenaire de liaison sous forme polymère.

9. Réactif pour la détermination d'un anticorps de la classe des immunoglobulines M et spécifique à un antigène, par un procédé selon les revendications précédentes, caractérisé en ce qu'en plus des additifs de test habituels pour les déterminations immunologiques, il contient des partenaires de liaison sous forme monomère et deux récepteurs R₁ et R₂ capables de se lier à l'anticorps à déterminer et dont le composant essentiel est chaque fois un partenaire de liaison sous forme polymère, R₁ étant capable de se lier à une phase solide et R₂ portant un marqueur.

10. Utilisation de partenaires de liaison sous forme monomère pour la désactivation d'anticorps IgG et/ou de facteurs rheuma dans la détermination d'un anticorps IgM spécifique à un antigène.
